(19) Europäisches Patentamt
European Patent Office
Office européen des brevets

(11) Veröffentlichungsnummer: **0 024 473**
**B1**

(12) # EUROPÄISCHE PATENTSCHRIFT

(45) Veröffentlichungstag der Patentschrift: **20.06.84**

(51) Int. Cl.³: **C 11 B 9/00,** C 07 D 321/10

(21) Anmeldenummer: **80102283.1**

(22) Anmeldetag: **28.04.80**

(54) **Verwendung einer Gruppe von Dioxa-alkyl-tricyclododecenen als Riechstoffe; diese enthaltende Riechstoffkompositionen; neue Dioxa-alkyl-tricyclododecene dieser Gruppe.**

(30) Priorität: **05.05.79 DE 2918168**

(43) Veröffentlichungstag der Anmeldung: **11.03.81 Patentblatt 81/10**

(45) Bekanntmachung des Hinweises auf die Patenterteilung: **20.06.84 Patentblatt 84/25**

(84) Benannte Vertragsstaaten: **AT BE CH DE FR GB IT LI NL**

(56) Entgegenhaltungen:
**GB - A - 869 485**
**LU - A - 78 181**
**US - A - 3 799 892**

**BULLETIN DE LA SOCIETE CHIMIQUE DE FRANCE, Juli-August 1975, Seiten 1763-1766, Paris, FR. J. SOULIER et al.: "Synthèse de dihydro-4,7-dioxépinnes-1,3 et de systèmes polycycliques contenant de noyau du dioxépanne-1,3 (Note de laboratoire)"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Conrad, Jens, Dr., Dürerweg 15, D-4010 Hilden (DE)**
Erfinder: **Upadek, Horst, Dr., Kempenweg 18 a, D-4006 Erkrath (DE)**
Erfinder: **Bruns, Klaus, Dr., Notburgaweg 6, D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, dass 4,6-Dioxa-5-alkyl-tricyclo-[7.21.$0^{2,8}$]-dodec-10-ene der allgemeinen Formel

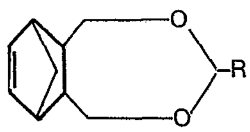

in der R einen gesättigten, geradkettigen oder verzweigten aliphatischen Kohlenwasserstoffrest mit 1–8 Kohlenstoffatomen darstellt, in vorteilhafter Weise als Riechstoffe in Kompositionen zur Parfümierung kosmetischer und technischer Präparate verwendet werden können.

Von besonderem Interesse ist dabei in parfümistischer Hinsicht das 4,6-Dioxa-5-isopropyl-tricyclo-[7.2.1.$0^{2,8}$]-dodec-10-en.

Die Herstellung zweier der erfindungsgemäss zu verwendenden Produkte ist bereits von J. Souliers, M. Farines, A. Bonafos-Bastouill und A. Laguerre im Bull. Soc. Chim. Fr. (1975) Seite 1763–66 bzw. im GB-A-869 485 beschrieben worden, ohne dass olfaktorische Eigenschaften der Verbindung erkannt worden wären. Gemäss nachfolgendem Schema wurden danach aus Cyclopentadien und Maleinsäurederivaten die entsprechenden Diels-Alder-Addukte hergestellt. Die Reduktion der Diels-Alder-Addukte mit Lithium-Aluminiumhydrid liefert die Diole, die durch Acetalisierung mit Aldehyden zu den Dioxepanen cyclisiert werden.

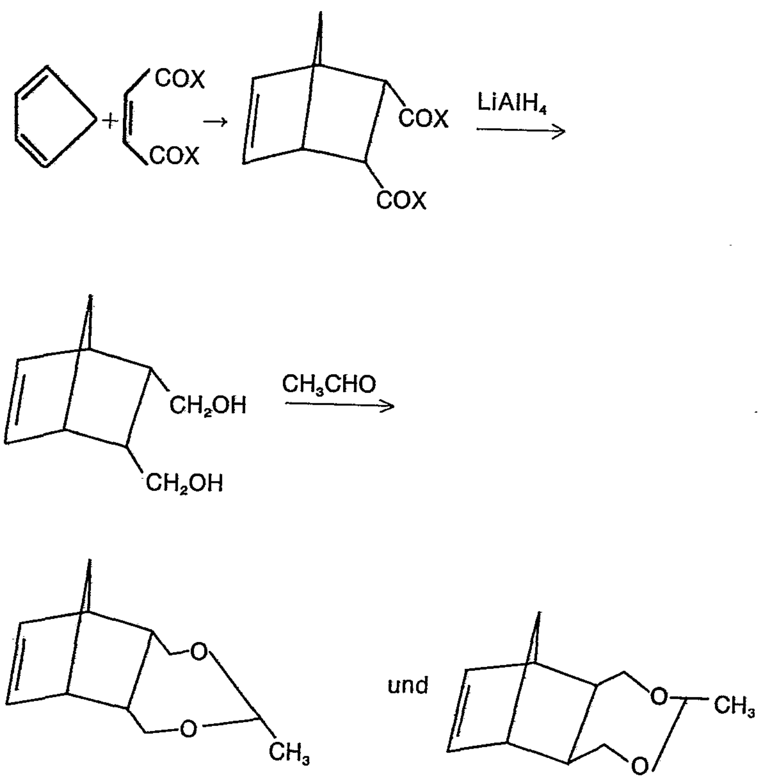


Die wie vorstehend angegeben in der Literatur beschriebene Herstellung des

4,6-Dioxa-5-methyl-tricyclo-[7.2.1.$0^{2,8}$]-dodec-10-en

ist jedoch für eine technische Herstellung nicht gangbar. Die technische Herstellung der erfindungsgemäss zu verwendenden

4,6-Dioxa-5-alkyl-tricyclo-[7.2.1.$0^{2,8}$]-dodec-10-ene

erfordert eine Umgehung der oben skizzierten Reduktionsstufe, die in befriedigender Weise nur mit Lithiumaluminiumhydrid zu den Diolen führt und daher besondere Sicherheitsmassnahmen erfordert.

Die erfindungsgemäss zu verwendenden

4,6-Dioxa-5-alkyl-tricyclo-[7.2.1.$0^{2,8}$]-dodec-10-ene

sind in zwei Reaktionsschritten leicht zugänglich. Im ersten Reaktionsschritt (a) werden die für die Adduktbildung benötigten 1,3-Dioxa-2-alkyl-5-cycloheptene nach üblichen Methoden der organischen Chemie durch Acetalisierung von Cis-2-Buten-1,4-diol mit aliphatischen Aldehyden hergestellt. (vgl. Bull. Soc. Chim. Fr. (1975) S. 1763). Im zweiten Verfahrensschritt (b) wird das nach (a) erhaltene Dioxepin mit Cyclopentadien, das zweckmässigerweise als Dimeres eingesetzt und bei Reaktionstemperaturen über 150 °C «in situ» gebildet wird, zur gewünschten Verbindung umgesetzt. (vgl. GB-A-869 485, S. 1, Zeile 57 ff.). Empfehlenswert ist die Zudosierung von Dicyclopentadien zum vorgelegten Dioxepin bei einer Reaktionstemperatur von zirka 200 °C. Nach destillativer Aufarbeitung wird das Produkt in der

Regel als Gemisch der vier möglichen Stereoixomeren erhalten und ohne weitere Trennung als Riechstoff eingesetzt. Die Reaktion verläuft nach folgendem Schema:

a) 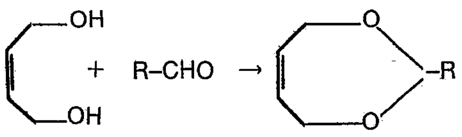

b) 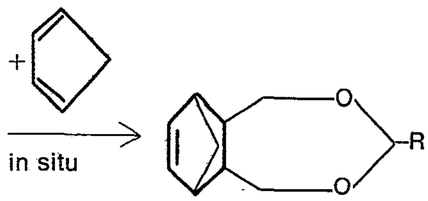


Von den erfindungsgemäss zu verwendenden unter die allgemeine Formel fallenden Produkten sind bisher das

4,6-Dioxa-5-methyl bzw. 4,6-Dioxa-5-isopropyl-tricyclo-[7.2.1.$0^{2,8}$]-dodec-10-en

als solches literaturbekannt, ohne dass jedoch seine Eignung als Riechstoff erkannt worden wäre.

Als erfindungsgemäss zu verwendende Produkte sind

4,6-Dioxa-5-methyl-, 4,6-Dioxa-5-ethyl-,
4,6-Dioxa-5-propyl-, 4,6-Dioxa-5-i-propyl-,
4,6-Dioxa-5-butyl-, 4,6-Dioxa-5-pentyl-,
4,6-Dioxa-5-hexyl-, 4,6-Dioxa-5-heptyl-,
4,6-Dioxa-5-octyl-tricyclo [7.2.1.$0^{2,8}$]-dodec-10-en

zu nennen.

Die erfindungsgemäss zu verwendenden 4,6-Dioxa-5-alkyl-tricyclo [7.2.1.$0^{2,8}$] dodec-10-ene sind wertvolle Riechstoffe mit charakteristischen Geruchsnoten. Sie lassen sich sehr gut zu neuartigen und interessanten Geruchsnuancen kombinieren. Unter den Produkten kommt dem

4,6-Dioxa-5-i-propyl-tricyclo [7.2.1.$0^{2,8}$] dodec-10-en

mit seiner fruchtigen, pilzigen, Zitrus-Jasmon-Note die grösste Bedeutung zu, da diese für die Entwicklung neuartiger Riechstoffkompositionen besonders geeignet ist.

Die erfindungsgemäss als Riechstoffe zu verwendenden

4,6-Dioxa-5-alkyl-tricyclo [7.2.1.$0^{2,8}$] dodec-10-ene

können mit anderen Riechstoffen in den verschiedensten Mengenverhältnissen zu neuen Riechstoffkompositionen gemischt werden. Im allgemeinen wird sich der Anteil der

4,6-Dioxa-5-alkyl-tricyclo [7.2.1.$0^{2,8}$] dodec-10-ene

in den Riechstoffkompositionen in den Mengen von 1 bis 50 Gewichtsprozent, bezogen auf die gesamte Komposition, bewegen. Derartige Kompositionen können zur Parfümierung von Kosmetika wie Cremes, Lotionen, Aerosolen, Toilettenseifen, technischen Artikeln wie Wasch- und Reinigungsmitteln, Desinfektionsmitteln und Textilbehandlungsmitteln dienen.

Die nachfolgenden Beispiele sollen den Gegenstand der Erfindung näher erläutern, ohne ihn jedoch hierauf zu beschränken.

Beispiele

Zunächst wird die Herstellung der erfindungsgemäss zu verwendenden

4,6-Dioxa-5-alkyl-tricyclo[7.2.1.0.$^{2,8}$]-dodec-10-ene

beschrieben, wobei es sich, wie vorstehend ausgeführt, um Gemische stereoisomerer Formen handelt.

A) Herstellung von 4,6-Dioxa-5-isopropyl-tricyclo-[7.2.1.$0^{2,8}$]-dodec-10-en

a) Herstellung von 1,3-Dioxa-2-isopropyl-5-cycloheptan.

Die Mischung aus 352 g (4 Mol) Cis-2-Buten-1,4-diol, 288 g (4 Mol) Isobutyraldehyd, 3,5 g p-Toluolsulfonsäure und 150 ml Toluol wurde 1 Stunde am Wasserabscheider gekocht, wobei 72 ml Wasser abgeschieden wurden und die Temperatur auf 134 °C anstieg. Die auf 40 °C abgekühlte Lösung wurde mit 13 g 40%iger wässriger Kaliumcarbonatlösung neutralisiert und im Vakuum destilliert. Es wurden 440 g, das sind 78% der theoretischen Ausbeute, 1,3-Dioxa-2-isopropyl-5-cyclohepten vom Siedepunkt 65 bis 85 °C bei 27 mbar erhalten.

b) 4,6-Dioxa-isopropyl-tricyclo [7.2.1.$0^{2,8}$]-dodec-10-en.

200 g (1,4 Mol) 1,3-Dioxa-2-isopropyl-5-cyclohepten wurden im Autoklaven auf 200 °C erhitzt, im Laufe von 2 Stunden mit 93 g (0,7 Mol) Dicyclopentadien versetzt und 3 Stunden nachgerührt. Das Rohprodukt wurde im Vakuum überdestilliert (30 bis 89 °C bei 0,0133 mbar, 239 g) und anschliessend fraktioniert. Es wurden 135 g, das sind 47% der theoretischen Ausbeute, an

4,6-Dioxa-5-isopropyl-tricyclo[7.2.1.$0^{2,8}$]dodec-10-en

vom Siedepunkt 95 °C bei 0,8 mbar und einem Brechungsindex $n_D^{25}$ = 1,4890 erhalten. Der Geruch der Verbindung war fruchtig, pilzig, erdig, luftig mit Zitrus-und Jasmon-Note.

Analog dem vorstehend beschriebenen Verfahren wurden die nachfolgend aufgeführten

4,6-Dioxa-5-alkyl-tricyclo[7.2.1.$0^{2,8}$]dodec-10-ene

hergestellt. Sie stellen gleichfalls farblose Öle oder Festprodukte dar.

B) 4,6-Dioxa-5-methyl-tricyclo[7.2.1.$0^{2,8}$]dodec-10-en.

Schmelzpunkt 92 bis 93 °C
Geruch: grün, Gurken-Note

C) 4,6-Dioxa-5-ethyl-tricyclo[7.2.1.$0^{2,8}$]dodec-10-en.

Schmelzpunkt 46 bis 65 °C
Geruch: Laub-, Humus-, Champignon-Note, frisch

D) 4,6-Dioxa-5-propyl-tricyclo[7.2.1.$0^{2,8}$]dodec-10-en.

Siedepunkt 62 bis 68 °C bei 0,0013 mbar, $n_D^{25}$ = 1,4921
Geruch: Laub-, Humus-, Champignon-Note, frisch

Beispiel 1 Lavendelöl, künstlich

| | Gewichtsteile |
|---|---|
| 4,6-Dioxa-5-propyl-tricyclo[7.2.1.0$^{2,8}$] dodec-10-en | 100 |
| Lavandinöl Abrialis | 400 |
| Lavandinöl acetyliert | 300 |
| Terpinylpropionat | 50 |
| Linalool | 40 |
| Linalylisobutyrat | 20 |
| Lavandulol | 20 |
| Ethylamylketon | 15 |
| Nerol | 10 |
| Spiköl | 10 |
| Linalylvalerat | 5 |
| Cumarin | 5 |
| Citronellol/Citronellylacetat | 5 |
| Geraniol/Geranylacetat | 5 |
| Linalooloxid | 5 |
| n-Hexylalkohol | 3 |
| 1-Octen-3-ol/Acetat | 3 |
| Cuminalkohol | 1 |
| Eugenol | 1 |
| Methylheptenon | 1 |
| Hexen-2-al (1) 10%ig | 1 |
| | 1000 |

Beispiel 2 Parfümierung für Seife

| | Gewichtsteile |
|---|---|
| 4,6-Dioxa-5-isopropyl-tricyclo-[7.2.1.0$^{2,8}$]dodec-10-en | 155 |
| Boisambrene(R), Henkel | 230 |
| Citronellol | 150 |
| Geraniol | 140 |
| Hydroxycitronellol | 80 |
| α-Amylzimtaldehyd | 60 |
| Phenylacetaldehyd 50% in DEP | 50 |
| Vetisclaron(R), Henkel | 60 |
| Isogeranylnitril | 30 |
| Phenylethylalkohol | 30 |
| Benzylacetat | 10 |
| Nonylaldehyd | 5 |
| | 1000 |

**Patentansprüche**

1. Verwendung von 4,6-Dioxa-5-alkyl-tricyclo-[7.2.1.0$^{2,8}$]-dodec-10-enen der allgemeinen Formel

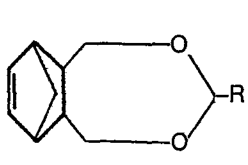

in der R einen gesättigten, geradlinigen oder verzweigten aliphatischen Kohlenwasserstoffrest mit 1 bis 8 Kohlenstoffatomen darstellt, als Riechstoffe.

2. Verwendung von 4,6-Dioxa-5-isopropyl-tricyclo-[7.2.1.0$^{2,8}$]-dodec-10-en nach Anspruch 1, als Riechstoff.

3. Riechstoffkompositionen, gekennzeichnet durch einen Gehalt an 4,6-Dioxa-5-alkyl-tricyclo-[7.2.1.0$^{2,8}$]-dodec-10-enen nach Anspruch 1 und 2.

4. Riechstoffkompositionen nach Anspruch 3, dadurch gekennzeichnet, dass sie die 4,6-Dioxa-5-alkyl-tricyclo-[7.2.1.0$^{2,8}$]-dodec-10-ene in einer Menge von 1 bis 50 Gewichtsprozent, bezogen auf die gesamte Komposition, enthalten.

5. 4,6-Dioxa-5-ethyl-tricyclo-[7.2.1.0$^{2,8}$]-dodec-10-en.

6. 4,6-Dioxa-5-propyl-tricyclo-[7.2.1.0$^{2,8}$]-dodec-10-en.

**Claims**

1. The use of 4,6-dioxa-5-alkyl-tricyclo-[7.2.1.0$^{2,8}$]-dodec-10-enes corresponding to the following general formula

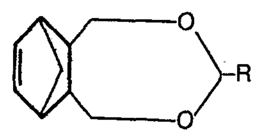

in which R represents a saturated, straight-chain or branched-chain aliphatic hydrocarbon radical containing from 1 to 8 carbon atoms, as perfumes.

2. The use of 4,6-dioxa-5-isopropyl tricyclo-[7.2.1.0$^{2,8}$]-dodec-10-ene as claimed in Claim 1, as a perfume.

3. Perfume compositions, characterized by a content of the 4,6-dioxa-5-alkyl tricyclo-[7.2.1.0$^{2,8}$]-dodec-10-enes claimed in Claims 1 and 2.

4. Perfume compositions as claimed in Claim 3, characterized in that they contain the 4,6-dioxa-5-alkyl tricyclo-[7.2.1.0$^{2,8}$]-dodec-10-enes in a quantity of from 1 to 50% by weight, based on the composition as a whole.

5. 4,6-dioxa-5-ethyl-tricyclo-[7.2.1.0$^{2,8}$)-dodec-10-ene.

6. 4,6-dioxa-5-propyl-tricyclo-[7.2.1.0$^{2,8}$]-dodec-10-ene.

**Revendications**

1. Utilisation de 4,6-dioxa-5-alcoyl-tricyclo-[7.2.1.o$^{2,8}$]-dodéc-10-ènes de formule générale:

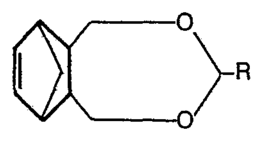

dans laquelle R représente un radical hydrocarboné aliphatique saturé à chaîne droite ou ramifiée ayant 1 à 8 atomes de carbone, en tant que matières odorantes.

2. Utilisation du 4,6-dioxane-5-isopropyl-tricyclo-[7.2.1.0$^{2,8}$]-dodéc-10-ène selon la revendication 1 comme matière odorante.

3. Compositions de matières odorantes, caractérisées par une teneur en 4,6-dioxa-5-alcoyl-tricyclo-[7.2.1.$0^{2,8}$]-dodéc-10-ènes selon les revendications 1 et 2.

4. Compositions de matières odorantes selon la revendication 3, caractérisées en ce qu'elles contiennent les 4,6-dioxa-5-alcoyl-tricyclo-[7.2.1.$^{2,8}$]-dodéc-10-ènes en une quantité de 1 à 50% en poids par rapport à la composition totale.

5. 4,6-dioxa-5-éthyl-tricyclo-[7.2.1.$0^{2,8}$]-dodéc-10-ène.

6. 4,6-dioxa-5-propyl-tricyclo-[7.2.1.$0^{2,8}$]-dodéc-10-ène.